# EUROPEAN PATENT APPLICATION

(11) **EP 3 769 770 A1**
(43) Date of publication of application: **27.01.2021**
(21) Application number: 19187921.2
(22) Date of filing: 23.07.2019
(51) Int. Cl.: A61K 35/17, C12N 5/0783, G01N 33/50

(54) **METHOD FOR INHIBITING OR PREVENTING INFECTION IN A TRANSPLANT RECIPIENT**

(71) Applicant: Kiadis Pharma Intellectual Property BV, 1105 BP Amsterdam (NL)
(72) Inventor: VELTHUIS, Jurjen, 1105 BP Amsterdam-Duivendrecht (NL)
(74) Representative: Ringeling, Patricia Louise

(57) **Abstract**

A method for inhibiting or preventing infection in a transplant recipient, the method comprising (i) providing T-cells from several potential donors; (ii) subjecting the T-cells from each donor to a separate 3^{rd} party reactivity test; (iii) determining the reactivity of each donor, whereby the donor with the highest reactivity is selected as the preferred donor for transplantation.

## Description

### Field of the invention

The present invention relates to a method for inhibiting or preventing an infection in a transplant recipient. In particular, for to a method for inhibiting or preventing an infection in a stem cell transplant recipient.

### Background of the invention

Infections are a major cause of disease and mortality after transplantation. Several methods for treating infections in a transplant recipient exist. Bacterial infections may be combatted with antibiotics, although antibiotics resistance is an increasing challenge. Viral infections may be combatted with antiviral drugs, such as ganciclovir and foscarnet. These drugs may have several side-effects, such as are neutropenia and anaemia. WO2014/099908 discloses another method for preventing HCMV infection in a transplant patient, viz. administering antibodies which bind to human CMV gH and human CMV Complex I, in four steps. Yet another method for treating infections in transplant patients is disclosed in EP21137992, which discloses a method comprising administering to a transplant recipient an effective amount of an inhibitor of the protein LSD1 or a monoamine oxidase inhibitor.

It would be desirable to have a convenient and reliable method for inhibiting or preventing infections in a transplant patient which are a minimal or no extra burden to the patient.

### Short description of the figures

**Fig. 1** Proliferation index of ATIR101 towards different stimuli. Comparison between patients who developed mild or moderate infections (black bars) and patients who developed severe infections (white bars).
**Fig. 2** 3^{rd} party proliferation indexes of all ATIR101 samples. The line indicates the median value.
**Fig. 3** Kaplan-Meier analysis for the time-to-severe infections. Comparison between ATIR101 batches with a proliferation index > the median value and batches with a proliferation index ≤ the median value.
**Fig. 4** Proliferation index of donor samples towards different stimuli. Comparison between patients who developed mild or moderate infections (black bars) and patients who developed severe infections (white bars).
**Fig. 5** 3^{rd} party proliferation indexes of all donor samples. The line indicates the median value.
**Fig. 6** Kaplan-Meier analysis for the time-to-severe infections. Comparison between donor samples with a proliferation index >the median value and donor samples with a proliferation index ≤ the median value.
**Fig. 7** Correlation between 3^{rd} part reactivity of donor cells and ATIR101 3^{rd} party reactivity.

### Detailed description

The present invention relates to a method for inhibiting or preventing an infection in a stem cell transplantation recipient for whom there are several potential donors available, the method comprising:
(i) providing a biological sample from several potential donors, each biological sample comprising donor T-cells;
(ii) subjecting the biological sample from each donor to a separate 3rd party reactivity test;
(iii) determining the 3rd party reactivity of each potential donor, whereby the potential donor with the highest 3rd party reactivity is selected as the preferred donor for stem cell transplantation.

One advantage of the method is that severe or clinically significant infections in a transplant recipient may be prevented or inhibited by carefully selecting a preferred transplant donor from several potential donors. Transplantations using the preferred donor lead to infections of less severity, and thus to a reduction in the number of severe infections.

Infection refers to the invasion or multiplication of microorganisms, such as bacteria, fungi or viruses, which are not normally present within an individual's body, or to the presence of such microorganisms in abnormal amounts. The infection is typically caused by or associated with the invasion or multiplication of microorganisms, such as bacteria or viruses, preferably viruses, such as viruses from the adenoviridae, herpesviridae, parvoviridae, picornaviridae, pneumoviridae or polyomaviridae family, including but not limited to adenovirus, cytomegalus virus (CMV), Epstein-Barr virus (EBV), herpes simplex viruses 1 (HSV-1), herpes simplex viruses 2 (HSV-2), polyomavirus, respiratory syncytial virus (RSV), rhinovirus or parvovirus. The virus may be a DNA or an RNA virus and is preferably a human virus. Microorganisms may be reactivated after having been dormant or silent in a host for several weeks, months or years. In the present context, such reactivations of microorganisms are included in the term infection.

An infection may be subclinical, with no symptoms, or it may be clinical, with symptoms. Infections with mild or moderate symptoms usually do not require hospitalisation. Infections with significant or severe symptoms usually require hospitalisation. Whether or not hospitalisation is required will also depend on the condition of the patient. In a preferred embodiment, the method is used for preventing or inhibiting severe infections in particular, severe infections in immunocompromised patients.

Severity of infection is preferably graded by National Cancer Institute Common Terminology Criteria for Adverse Events Version 4.0, whereby: Grade 0 denotes no infection, Grade 1 and 2 are used for subclinical infections or infections with mild to moderate symptoms and Grades 3-5 are used for infections with severe or medically significant symptoms for which hospitalisation is typically indicated. Grade 3 infections require hospitalisation or invasive intervention, transfusion, elective interventional radiological procedure, therapeutic endoscopy or operation, but not life-threatening. Grade 4 is life-threatening or disabling. Grade 5 is death related to the infection. Using the method of the invention, in particular significant or severe infections, i.e. of Grade 3-5 severity, may be reduced and thereby non-relapse mortality (NRM) may be reduced. NRM is defined as death due to causes other than disease relapse or progression or other causes unrelated to the transplantation procedure, such as accident or suicide. In a preferred embodiment, the method is used for preventing or inhibiting Grade 3-5 infections in particular, Grade 3-5 infections in immunocompromised patients.

Due to the method of the invention, occurring infections are of less severity. In particular, severe infections are prevented or inhibited. Prevention may be apparent from no infections being present, in particular no Grade 3-5 infections being present. Inhibition may be apparent from less infections being present, in particular less Grade 3-5 infections being present. Prevention or inhibition of infection may be complete or partial, such as an inhibition leading to at least 10%, at least 20%, at least 30%, at least 40%, at least 50%, at least 60% or at least 80% less infections; it may be permanent or temporary, for example at least 30 days, at least 4 months or at least 6 months, more preferably at least 1 or 2 years, such as from 6 months to 1 year or from 1 year to 2 years and is preferably permanent and complete. In one embodiment, the chance of developing within one year an infection with Grade 3 -5 severity is reduced with at least 10%, at least 20%, at least 30%, at least 50%, at least 60% or at least 80% if the donor has high 3^{rd} party reactivity in comparison to the other available donors, e.g. below or at the median of the group of available donors. In another embodiment, if the donor has high 3^{rd} party reactivity in comparison to the other available donors, e.g. above the median of the group of available donors, the number of infections with Grade 3-5 severity occurring within one year is less than 50%, less than 40%, less than 30%, less than 20%, less than 15% or less than 10% of the number of infections with Grade 3-5 severity associated with a donor with low 3^{rd} party reactivity, e.g. below or at the median of the group of available donors. Most preferably, the inhibition of microorganisms is complete and permanent and detectable infectious microorganisms are reduced to zero. Methods for identifying and quantifying viruses, such as CMV and EBV, are known in the art and include immunological and nucleotide based methods, such as quantitative polymerase chain reaction. Quantification may be regularly, for example weekly, monthly or anything in between.

The recipient is preferably a patient who has received or will receive stem cell transplantation, preferably hematopoietic stem cell transplantation, such as a patient with acute myeloid leukemia (AML), acute lymphatic leukemia (ALL), chronic myeloid leukemia (CML), chronic lymphatic leukemia (CLL) Hodgkin or non-Hodgkin lymphoma, multiple myeloma, myelodysplastic syndrome (MDS), sickle cell anaemia, severe aplastic anaemia (SAA), granulomatosis or thalassemia. Donor and recipient may be of any species, preferably they are mammals. Preferably, the recipient is a human being, more preferably, both donor and recipient are human beings.

In one embodiment, the transplantation is an allogeneic hematopoietic stem cell transplantation. The stem cell transplantation may be part of the treatment of immunological disorders or blood disorders, which may be inherited, such as blood cancers or blood disorders, including acute myeloid leukemia (AML), acute lymphatic leukemia (ALL), chronic myeloid leukemia (CML), chronic lymphatic leukemia (CLL) Hodgkin or non-Hodgkin lymphoma, multiple myeloma, myelodysplastic syndrome (MDS), sickle cell anaemia, severe aplastic anaemia (SAA), granulomatosis or thalassemia.

The method according to the invention is used when several potential donors are available for the recipient and a selection has to be made between the several potential donors. The several potential donors may include matched donors (HLA identical to the recipient), such as matched siblings, and haploidentical donors, meaning that donor and recipient are of non-identical (half-matched) HLA haplotype and have mismatches at one or more loci of the unshared haplotype, such as may be the case for example for parents, children or siblings of the recipient. In one embodiment, the haploidentical donor has two or three mismatches at the HLA-A, HLA-B or HLA-DR loci of the unshared haplotype. In contact, the mismatches will lead to donor cells being activated by mismatched HLA antigens on the recipient cells. In one embodiment, the pool of several potential donors consists of haploidentical donors.

Each biological sample comprises donor T-cells from one single donor. The donor T-cells (T-lymphocytes) in the biological sample may be isolated T-cells or, preferably, T-cells in a peripheral blood mononuclear cells (PBMCs) fraction. The fraction of T-cells in the biological sample may be at least 30%, at least 40%, at least 50% or at least 60%, such as between 40% and 99%, between 40% and 60% or between 50% and 90%, all percentages based on the total number of cells in the biological sample. In one embodiment, the biological sample comprises between 40% and 60% of T-cells and less than 20% NK-cells and less than 20% B-cells, all percentages based on the total number of cells in the biological sample. Both CD4+ and CD8+ T-cells may be present in the biological sample. According to the method of the invention, the biological sample of each potential donor is kept separately from the other potential donors. For the biological sample of each potential donor a reactivity profile is determined, which is a measure for the activation of T-cells. The activation of T-cells may be apparent from upregulation of certain surface markers, e.g. upregulation of the IL-2 receptor or subunits thereof, such as the IL-2 receptor alpha chain, also referred to as CD25, or from proliferation, i.e. dividing and multiplication. The reactivity profile may be measured in any suitable way, for example by measuring cytokine production or by measuring proliferation. The reactivity profile of at least 2, at least 5 or at least 10 potential donors may be determined for selection of the one with the highest 3^{rd} party reactivity, such as the highest proliferation index or the highest cytokine production.

In one embodiment, the reactivity profile is measured by proliferation. Proliferation may require proliferation factors such as for example interleukins, such as interleukin 2, or other growth factors and may be measured in a proliferation assay.
Suitable proliferation inducing stimuli may include:
1. irradiated autologous donor cells, to determine baseline proliferation by addition of cells that may provide a 'feeder effect'. Feeder cells release nutrients and provide for matrix support;
2. irradiated recipient cells, to determine the (retained) reactivity against the recipient, which reactivity is preferably as low as possible;
3. irradiated 3rd party cells, to determine the (retained) reactivity against unrelated HLA-antigens, which reactivity is preferably as high as possible;
4. CD3/28 coated beads to generate a pan T-cell stimulus.

Any suitable proliferation assay may be used. In one embodiment, the proliferation assay uses cells loaded with carboxyfluorescein diacetate succinimidyl ester (CFSE). In this assay, cell cultures are initiated by addition of different proliferation-inducing stimuli for five days in the presence of 1 IU/ml human interleukin-2 in X-VIVO™15 medium with 10% v/v pooled human heat-inactivated PBMCs form different individuals. The proliferation (i.e. dilution of CFSE) of the cells may be analyzed using Modfit LT™ software (VSH software) returning a proliferation index (PI) which reflects the increase in cell number over the course of the assay. A PI of 1.0 represents no proliferation.

The selected donor may be a donor of stem cells or of a cell preparation as adjunctive therapy, which may comprise one single cell type or a mixture of cell types, such as hematopoietic stem cells, PBMCs, white blood cells, NK- cells, T-lymphocytes (T-cells). In one embodiment, the donor is a donor of hematopoietic stem cells and of an adjunctive cell preparation, such as an allo(recipient)reactive T- cell depleted mixed lymphocyte reaction product.

In the present context, a mixed lymphocyte reaction (MLR) refers to an ex vivo reaction wherein cell preparations from two individuals, at least one of them containing T-lymphocytes, are mixed to allow a proliferative response of the T- cells from one individual (the responder cells) to cells from another individual (the stimulator cells). For example, it may be used to see how cells of a donor react to cells of a prospective recipient of donor material. In such a case, the recipient cells are the stimulator cells. It may also be used in a process to make donor material suitable for a recipient.

The MLR may be any type of MLR. The MLR is preferably a one-way MLR, wherein the stimulator cells are treated to render them non-proliferative. Cells may be rendered non-proliferative in any suitable way, for example by interfering with their DNA functionality, such as by gamma irradiation, by treating them with mitomycin C or by fixating the cells, e.g. by formaldehyde or paraformaldehyde. The T-cells for MLR may be may be present in a preparation which comprises or consists of T cells. In one embodiment, the T-cells used in the MLR are present in a peripheral blood mononuclear cell (PBMC) fraction. The person skilled in the art knows how to prepare a PBMC fraction, e.g. by Ficoll®- gradient density centrifugation.

In the MLR product, activated or proliferating responder T- cells (alloreactive T-cells) may have been inactivated or destroyed to make the selected donor material suitable for the recipient. This may involve photodynamic photodynamic treatment, for example as described in EP 1 267 931 or in EP 3 258 968, using a rhodamine derivative, such as TH9042. Such alloreactive T- cell depleted mixed lymphocyte reaction products may be used in methods for treating or preventing graft versus host disease (GvHD), for example after or in combination with a haploidentical hematopoietic stem cell transplantation (HSCT), in particular after or in combination with T cell depleted CD34+ HSCT. In another embodiment, the depletion of such alloreactive T-cells occurs through the *in vivo* or *ex vivo* administration of cyclophosphamide.

In another aspect, the present invention relates to a kit which may conveniently be used for the method according to the invention. The kit comprises (i) a container comprising a reactivity assay reagent; (ii) a container comprising a mixture of cells from unrelated individuals or other reagents mimicking 3^{rd} party reactivity; and optionally, (iii) a container for incubation of the test samples and the 3^{rd} party cells or reagents. In one embodiment, the reactivity assay reagent is a reagent for a proliferation assay, for example the CFSE proliferation assay.
Embodiments, preferred embodiments and examples mentioned for the method are also applicable to the kit.

### Examples

### Materials and Methods

### Cell Collection and Donor Plasma Processing

Donors and patients diagnosed with acute myeloid leukemia (AML) or acute lymphatic leukemia (ALL) underwent mononuclear cell-enriched leukapheresis to obtain cells and plasma for a mixed lymphocyte reaction (MLR). Donor plasma was collected during leukapheresis, filtered with a 0.22 µm filter, and heat-inactivated at 56 °C for 60 mins.

### Mixed lymphocyte reaction (MLR)

Leukapheresis-obtained cells were washed and processed by automated mononuclear cell purification using a Sepax device (Biosafe, Eysins, Switzerland), following the standardized Sepax NeatCell protocol. Subsequently, donor peripheral blood mononuclear cells (PBMCs; 1×10⁶/mL) were co-cultured with gamma-irradiated (50 Gy) recipient PBMCs 1:1 in X-VIVO 15TM medium (Lonza, Basel, Switzerland), containing 2% donor plasma and 50 IU/mL interleukin-2 (proleukin, Novartis), for 4 days under static conditions.

### Photodynamic Treatment (PDT)

After the MLR, cells were centrifuged and pooled in X-VIVO 15TM containing 2.5% donor plasma and 5 µM TH9402 (Kiadis Pharma, Amsterdam) and incubated at 37°C, 5% CO₂. After 40 mins of coloration, cells were centrifuged (500 g, 15 mins) to remove coloration medium. The cell pellet was resuspended in X-VIVO 15TM with 10% donor plasma and incubated at 37°C, 5% CO₂ for 90 mins to allow extrusion of the dye. After extrusion, cells were immediately placed on the PDT device (Kiadis Pharma, Amsterdam) and exposed to visible light until an energy dose of 5 J/cm² was delivered. Cells were then collected and centrifuged (500 g, 15 mins) and the percentage of viable T cells in the total cell population determined by flow cytometry. The cell suspension volume required to formulate the product and test and retain samples (2×10⁶ cells/kg bodyweight) was withdrawn and centrifuged (500 g, 15 mins); cells were resuspended in saline with 20% donor plasma. From this suspension, 10 mL was transferred to the final product bag. Next, 10 mL pre-cooled (2-8 °C) dimethyl sulfoxide (DMSO)-containing medium with 40% donor plasma and 20% DMSO was added to the suspension in the final product bag, and the product was frozen until administered to the patient. Immediately thereafter, DMSO-containing medium was added 1:1 to the remaining suspension and frozen formulating samples required for further quality control and characterization assays.

### Carboxyfluorescein Succinimidyl Ester (CFSE)-dilution-based Proliferation Assay

A CFSE-dilution-based proliferation assay was used to determine the specificity of depletion of alloreactive T cells and the potency of remaining cells. Briefly, 2 µM CFSA-SE (Invitrogen Life Technologies, Bleiswijk, the Netherlands) was added to 2×10⁶ viable T cells/mL. The cells were incubated at 37 °C for 7 mins in the dark before addition of cold X-VIVO 15TM with 10% plasma and incubation at 2-8 °C for 5 mins in the dark to quench the labeling. Cells were then washed three times by centrifuging at 500 g for 7 mins at RT. Next, the cells were resuspended in CFSE culture medium (X-VIVO 15TM with 10% plasma and 1 U/mL interleukin-2) to a concentration of 1×106 viable cells/mL.

Co-cultures were set up using 1×10⁵ cells/well at 1:1 labeled T cell:stimulus ratio. Four different proliferation-inducing stimuli were used: irradiated autologous donor cells, irradiated recipient cells, irradiated third-party cells, and anti-CD3/CD28 beads. Cells were incubated at 37°C, 5% CO2 for 5 days; the proliferation pattern was then determined using Modfit LT-analysis (VSH Software).

### Infections

Infections were clinically apparent infectious disease with symptoms or a viral reactivation as determined by polymerase chain reaction. Each infection with a different infectious agent is one event. Several infections with the same infectious agent, e.g. EBV, are counted as one event, unless the infection has been resolved for longer than a month. Infections are graded in accordance with CTCAE vs. 4.0. Allocation of events to assessment periods is based on the onset date of the respective infection.

### Statistics

The Wilcoxon signed-rank test was used to compare characteristics of ATIR101 batches. The statistical significance level was set at P≤0.05. Rates of time-to-event endpoints were calculated using the log-rank test Statistical analyses on the characteristics of ATIR101 were performed with GraphPad Prism v7. SAS software (v9.3) was used for the remaining analyses.

### Example 1

A mixed lymphocyte reaction (MLR) followed by photodynamic treatment (PDT) was performed with donor PBMCs and irradiated-recipient cells using TH9402. MLR and PDT were carried out as described under Materials & Methods. The PDT inactivated or destroyed the recipient reactive donor T-cells present in the MLR mixture. The resulting product was the ATIR101 product for adjunctive infusion into 23 stem cell recipients, aged between 18-65 years, without a suitable matched donor, 28 days (mean value) after HSCT. Each donor was haploidentical to the respective recipient. The haploidentical donor of each recipient was subjected to a 3^{rd} party proliferation test to determine a proliferation index (PI).

Two years after HSCT, 50% of the surviving recipients had developed no infections or mild or moderate infections (Grade 0-2) and about 36% had developed infections of Grade ≥3, as presented in Table 1. For each patient, the worst severity of infection is shown. Severity was graded according to CTCAE vs. 4.0. Allocation of events to assessment periods is based on the onset date of the respective infection. N reflects the number of patients at the start of the assessment period and n reflects the number of events in a certain period, which may be in the same person. Infections with unknown causative agent are not included in this table.

**Table 1**

| | **From HSCT to ATIR101 infusion (N=23)** | **From ATIR101 infusion to 6 months after HSCT (N=23)** | **From 6 months to 1 year after HSCT (N=19)** | **From 1 year to 2 years after HSCT (N=14)** |
|---|---|---|---|---|
| All Grades, n (%) | 9 (39.1) | 16 (69.6) | 12 (63.2) | 12 (85.7) |
| Grade 1/2, n (%) | 9 (39.1) | 9 (39.1) | 9 (47.4) | 7 (50.0) |
| Grade ≥3, n (%) | 0 | 7 (30.4) | 3 (15.8) | 5 (35.7) |
| n= number of events in a certain period; | | | | |
| N= number of patients | | | | |

There were 20 events of severe viral infections or reactivations (Table 2). Of these severe viral infections or reactivations 85% were Grade 3. There was 1 Grade 4 event (EBV) and there were 2 deaths (Grade 5 JC virus-mediated encephalopathy and adenovirus infection).

**Table 2**

| **Grade ≥3 viral infections** | **All Grade ≥3** | **Grade 3** | **Grade 4** | **Grade 5** |
|---|---|---|---|---|
| EBV | 5 | 4 | 1 | 0 |
| CMV | 5 | 5 | 0 | 0 |
| Herpes | 3 | 3 | 0 | 0 |
| Polyomaviruses | 2 | 1 | 0 | 1 |
| Respiratory syncytial virus | 1 | 1 | 0 | 0 |
| Adenovirus | 2 | 1 | 0 | 1 |
| Rhinovirus | 1 | 1 | 0 | 0 |
| Parvovirus | 1 | 1 | 0 | 0 |
| **Total number of events** | 20 | 18 | 1 | 2 |

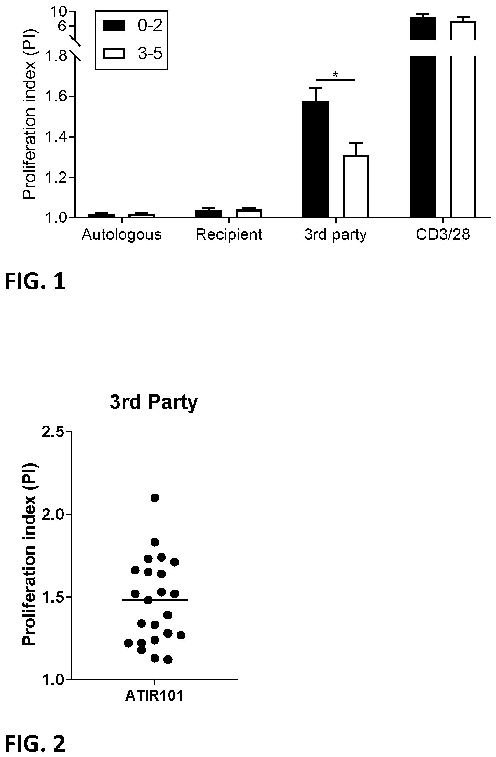

The reactivity of ATIR101 given to recipients who had developed no infection or an infection with severity Grade 0-2 after one year was compared to the reactivity of ATIR101 given to recipients who had developed significant infections (Grade 3-5) after one year. The results are presented in Fig. 1. The asteriks (*) indicates a statistically significant difference.
The results show that the reactivity of ATIR101 towards 3^{rd}-party antigens was significantly lower in batches administered to patients who later developed Grade 3-5 infections, compared with patients who later developed a Grade 0-2 infection. Next, the 3^{rd} party proliferation indexes of all ATIR101 samples were plotted (Figure 2); the line identifies the median.

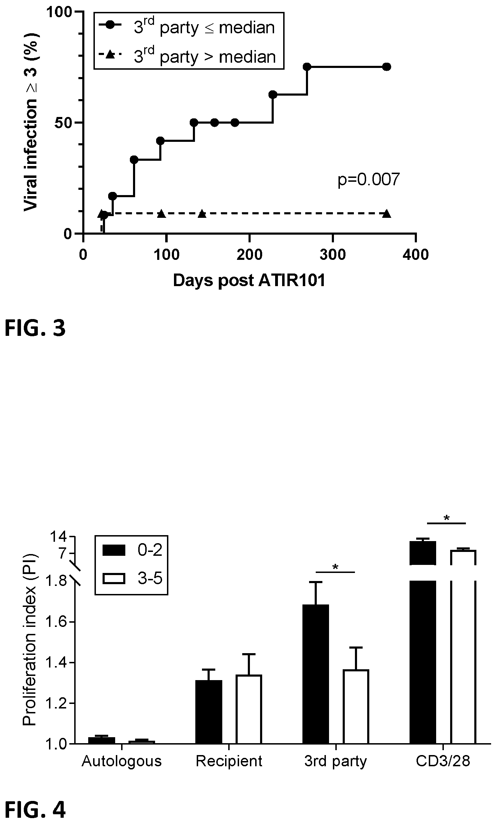

A time-dependent analysis was done using the Kaplan-Meier method for the time to viral infections Grade ≥3 in the batches above the median, and those under and including the median in Figure 2. Ten percent (10%) of patients who had received ATIR101 with a 3^{rd} party PI above the median, developed a Grade 3-5 infection within a year, whereas 80% of patients who had received ATIR101 with a 3^{rd} party PI up to and including the median, developed a Grade 3-5 infection within a year. Thus, patients receiving ATIR101 with a higher 3rd-party responsiveness were much less likely to develop a Grade 3-5 infection within a year than those with lower 3^{rd} party PI (Figure 3).

### Example 2

Donors whose ATIR101 was administered to patients who later developed Grade 0-2 viral infections appeared to have a significantly increased overall and 3rd-party reactivity (Figure 4; P<0.05), compared to those of patients with Grade 3-5 infections. The asteriks (*) indicates a statistically significant difference.

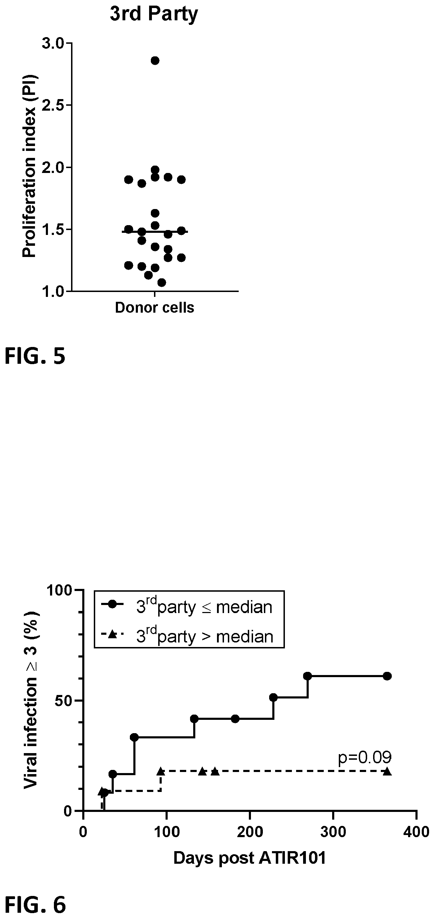

Next, the 3^{rd} party proliferation indexes of the donor cells were plotted, as shown in Figure 5. The line identifies the median. When donors displayed a high PI in response to 3rd-party stimulation (above the median), a lower rate of Grade ≥3 viral infections were observed, in comparison to donors with low PI (P=0.09) (Figure 6).

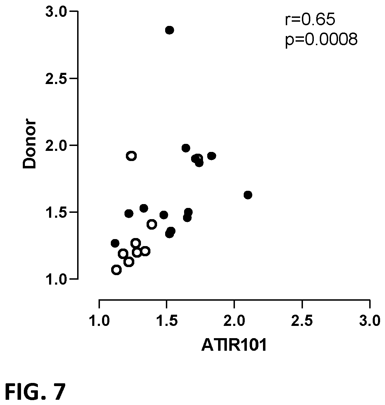

It was found that 3^{rd}-party reactivity of donor cells correlated with ATIR101 reactivity (Figure 7; P=0.0008), strongly indicating the importance of donor reactivity for the prevention of severe infections. Most patients with Grade ≥3 viral infections (open circles) were clustered to the bottom left of the plot, indicating lower reactivity in both donor and subsequently ATIR101, compared with patients without Grade ≥3 viral infections.

The results show that donor 3^{rd}-party reactivity is important for the prevention of severe infections. Increased reactivity to unrelated antigens leads to superior anti-viral immunity. Therefore donors with the highest 3^{rd}-party reactivity are preferred donors in case of multiple donors being available.

## Claims

1. A method for inhibiting or preventing an infection in a stem cell transplantation recipient for whom there are several potential donors available, the method comprising:
(i) providing a biological sample from several potential donors, each biological sample comprising donor T-cells;
(ii) subjecting the biological sample from each donor to a separate 3^{rd} party reactivity test;
(iii) determining the 3^{rd} party reactivity of each potential donor, whereby the potential donor with the highest 3^{rd} party reactivity is selected as the preferred donor for stem cell transplantation.

2. A method according to claim 1, wherein the transplantation is a hematopoietic stem cell transplantation, preferably a haploidentical hematopoietic stem cell transplantation.

3. A method according to any preceding claim, wherein the T-cells are present in a peripheral blood mononuclear cells (PBMC) preparation.

4. A method according to any preceding claim, wherein the stem cell transplantation is a T-cell depleted stem cell transplantation.

5. A method according to any preceding claim, wherein the T cell depleted stem cell transplantation is a CD34+ stem cell transplantation.

6. A method according to any preceding claim, wherein the stem cell transplantation is a graft containing CD34+ stem cells and T-cells.

7. A method according to any preceding claim, wherein the 3^{rd} party reactivity of each potential donor is determined by a T-cell proliferation assay.

8. A method according to any preceding claim wherein the donor is a donor for stem cells.

9. A method according to any preceding claim wherein the donor is a donor for T-cells or NK cells.

10. A method according to claim 9, wherein the T-cells are depleted of recipient-reactive T-cells.

11. A method according to claim 9 or 10, wherein the NK cells are enriched in recipient reactive NK-cells.

12. A method according to any preceding claim, wherein the infection is a viral infection, such as an infection of cytomegalovirus, herpes virus, polyomavirus, respiratory syncytial virus, adenovirus or rhinovirus or parvovirus,

13. A method according to any preceding claim, wherein the viral infection is of grade 3 or higher.

14. The use of high 3^{rd} party reactivity as positive criterium for selection of a donor.

15. The use according to claim 14, wherein the proliferaton index of the donor cells is the 3^{rd} party reactivity.
